# EUROPEAN PATENT APPLICATION

(11) **EP 4 755 206 A1**
(43) Date of publication of application: **10.06.2026**
(21) Application number: 24217251.8
(22) Date of filing: 03.12.2024
(51) Int. Cl.: A24B 13/00, A24B 15/30, A61K 31/465

(54) **SMOKELESS ARTICLE**

(71) Applicant: Imperial Tobacco Limited, Bristol BS3 2LL (GB)
(72) Inventor: DE DOMINICIS, Mattia, Bristol, BS3 2LL (GB); JONES, Jill, Bristol, BS3 2LL (GB); ELLIS, Paul, Bristol, BS3 2LL (GB); OSBORNE, Victoria Lee, Bristol, BS3 2LL (GB)
(74) Representative: Elkington and Fife LLP

(57) **Abstract**

A smokeless article for oral delivery of a nicotinic component. The smokeless article comprises a first compartment and a second compartment, which enclose a first content and second content, respectively. The first and second content are prevented from passing between compartments by a barrier. The pH of the first content is different to the pH of the second content, and one of the contents comprise a nicotinic component. A method of manufacturing a smokeless article

## Description

### FIELD

The present disclosure relates to a smokeless article. In particular, the disclosure relates to a smokeless article for oral delivery of a nicotinic component. This disclosure also relates to methods of manufacturing the smokeless articles, kits comprising the smokeless articles and use of the smokeless articles.

### BACKGROUND

Smokeless articles are placed in the mouth where saliva extracts the soluble element from the content contained within. Typically, the smokeless article is placed in the oral cavity, sublingually or in the oral vestibule (between the teeth and lips/cheeks). The user may assist extraction by oral manipulation, such as by chewing and/or sucking or pressing on the outside of the mouth to squeeze the pouch.

There is a need to improve the delivery of the components of the content contained within the smokeless article to improve user perception and experience.

The present disclosure has been devised in light of the above considerations.

### SUMMARY

At its most general, the present disclosure relates to a smokeless article for oral delivery of a nicotinic component.

In a first aspect the present disclosure provides a smokeless article for oral delivery of a nicotinic component comprising: a water-permeable layer defining at least a first compartment and a second compartment; wherein the first compartment encloses a first content having a first pH, and the second compartment encloses a second content having a second pH; and wherein the first and second compartments are separated by a barrier which prevents passage of the first and second content between the first and second compartments; wherein the first pH is different to the second pH; and wherein at least one of the first and second content comprises a nicotinic component.

As used herein, a "smokeless article for oral delivery of a nicotinic component" is an article that does not need to be heated or burned in order to function. The smokeless article is for oral consumption, as used herein, the term "oral consumption" is intended to refer to any oral administration route achieved by placing the smokeless article into the oral cavity. This includes, but is not limited to, buccal, sublingual, periodontal, gingival and ingestion.

Without wishing to be bound by theory, it is thought that the provision of a first and second compartment allows the environment in each compartment to be tuned to improve the performance of the first and second contents contained therein. Providing two compartments enclosing first and second contents with different pH values allows, for example, components that perform better at a first pH and components that perform better at a second pH to be formulated in a single smokeless article by enclosing them separately in the first and second compartments. In this way, the invention allows any desired component (for example, a flavouring or an active ingredient) to perform better, ultimately improving user experience, or to improve storage by keeping incompatible components physically separated.

The smokeless article comprises first and second compartments which enclose first and second contents, respectively. In some examples the compartments are sealed, and in this way completely enclose their respective contents. In some examples the compartments each define an internal volume in which the first and second contents reside. In some examples the compartments enclose their respective contents such that the contents cannot leave the compartments (and leave the smokeless article), when the smokeless article is not in use (i.e. in storage), or when it is in use and, for example, scatter in the oral cavity.

In some examples, each of the first and second content independently occupies substantially all of the internal volume of the first and second compartments, respectively. Each of the first and second contents may occupy 80%, 85%, 90%, 95% or 100% of the internal volume of the first and second compartment, respectively.

The smokeless article comprises a water-permeable layer defining the exterior walls of the first and second compartments. In use, the water-permeable layer allows water from saliva to enter the smokeless article, resulting in extracting of one or more components from the first and second contents within the smokeless article.

In some examples, the water-permeable layer is porous. In some examples, at least 50% of the pores have a diameter of 50 µm to 200 µm, such as 100 µm to 175 µm or 125 µm or 150 µm. In some examples, at least 50% of the pores have a diameter of at least 100 µm. For example, in some examples at least 55%, 60%, 65%, 70%, 75%, 80%, 85%, 90%, 95% or 100% of the pores have such diameters.

In some examples the smokeless article may be formed from one or more materials. In some examples, the water permeable layer may be formed from one or more of fibre, paper, cloth and fabric. In some examples, the water-permeable later may be formed from one or more polymeric materials. In some examples, the polymeric material may be selected from one or more of hydroxypropyl cellulose (HPC), hydroxypropyl methylcellulose (HPMC), polyvinyl alcohol (PVOH), polyvinylpyrrolidone (PVP), polyethylene oxide (PEO) hydroxyethyl cellulose (HEC), polyethylene glycol (PEG), pullulan, sodium alginate, xanthan gum, tragancanth gum, guar gum, acacia gum, arabic gum, polyacrylic acid, maltodextrin, methylmethacrylate copolymer, carboxyvinyl copolymers, starch and gelatin.

The first and second content have a first and second pH, respectively. The pH of a content results from the chemical nature of the components of that content.

Without wishing to be bound by theory, the pH of a content is thought to affect nicotine absorption through the oral mucosa. It is important for smokeless articles to provide good nicotine absorption for a good user experience and good nicotine perception. In addition, it is preferred that the pH does not vary significantly over time before use, as this can reduce the shelf-life of a smokeless article.

For the user to extract the nicotine and, for example, get good absorption of nicotine through the oral mucosa, the pH of a content is preferably within a range allowing it to easily cross the mucous membranes. For instance, under acidic conditions, nicotine is protonated and does not readily cross mucous membranes

However, balanced against this is the fact that other desired components of a smokeless article might respond negatively to a pH that is desired for optimal nicotine absorption and perception.

The overall pH of a smokeless article or the pH of a content material may be determined by, for example, (i) placing the smokeless article or content material in 10 mL of distilled water (ii) agitating the mixture for at least 5 minutes and (iii) measuring the pH of the solution with a pH probe.

In some examples, the content maintains a stable pH (e.g. changing by less than about 0.5 pH units, preferably less than about 0.25 pH units) for at least 4 months, at least 5 months, at least 6 months, at least 7 months, or at least 8 months.

The smokeless article comprises a barrier separating the first and second compartments. The barrier prevents physical passage of the first and second content between the first and second compartments. It may be that the barrier prevents physical passage of the first and second solid content between the first and second compartments. It may be that the barrier prevents physical passage of the first and second content between the first and second contents before the smokeless article is used (i.e. before being exposed to saliva).

In some examples the barrier comprises a weld between two or more surfaces of the water-permeable layer. In some examples the weld is a heat weld. In some examples the barrier comprises a layer of material, for example a water permeable material as described herein, that prevents passage of the first and second contents between first and second compartments.

The barrier functions to prevent physical mixing of the first and second contents. In this way, the first and second compartments, when not in use or when in use, possess their own chemical environment, featuring different pH levels.

At least one of the first and second content comprises a nicotinic component. In some examples, the nicotinic component comprises nicotine freebase, a nicotine salt(s), a nicotine complex(es), a nicotine solvate(s), or mixtures thereof. In some examples, nicotine salt is selected from the group consisting of nicotine hydrochloride, nicotine dihydrochloride, nicotine monotartrate, nicotine ditartrate, nicotine ditartrate dihydrate, nicotine sulfate, nicotine zinc chloride monohydrate, nicotine salicylate and mixtures thereof.

In some examples, the smokeless article may comprise the nicotinic component in an amount of from 0.3 wt.% to 2.4 wt.%, from 0.4 wt.% to 2 wt.%, from 0.5 wt.% to 2 wt.%, from 0.5 wt.% to 1.9 wt.%, from 0.8 wt.% to 1.8 wt.%, from 1 wt.% to 1.6 wt.%, from 1.2 wt.% to 1.6 wt.%, from 1.3 wt.% to 1.5 wt.%, from 0.3 wt.% to 1.6 wt.%, from 0.4 wt.% to 1.6 wt.%, from 0.5 wt.% to 1.6 wt.%, from 0.5 to 1.5 wt.%, from 0.8 wt.% to 1.4 wt.%, or from 1.0 wt.% to 1.2 wt.% based on the total weight of the smokeless article, or based on the total weight of all content present, in all compartments of the smokeless article. In some examples, the content of the smokeless article that comprises the nicotinic component may comprise the nicotinic component in an amount of from 0.3 wt.% to 2.4 wt.%, from 0.4 wt.% to 2 wt.%, from 0.5 wt.% to 2 wt.%, from 0.5 wt.% to 1.9 wt.%, from 0.8 wt.% to 1.8 wt.%, from 1 wt.% to 1.6 wt.%, from 1.2 wt.% to 1.6 wt.%, from 1.3 wt.% to 1.5 wt.%, from 0.3 wt.% to 1.6 wt.%, from 0.4 wt.% to 1.6 wt.%, from 0.5 wt.% to 1.6 wt.%, from 0.5 to 1.5 wt.%, from 0.8 wt.% to 1.4 wt.%, or from 1.0 wt.% to 1.2 wt.% based on the total weight of that content.

In some examples, the total content of nicotinic component per smokeless article is from 4 to 15 mg. In some examples, the content of nicotinic component per smokeless article is about 7-10 mg. In some examples the content of nicotinic component per smokeless article is about 8 mg.

In some examples, the amount of water in the smokeless article (i.e. the total amount in all present contents) is from 30 wt.% to 60 wt.%, from 30 wt.% to 55 wt.%, from 35 wt.% to 60 wt.%, from 35 wt.% to 55 wt.%, from 30 wt.% to 50 wt.%, or from 30 wt.% to 45 wt.% based on the total weight of content.

In some examples, the first and second contents have a shelf-life (for example, measured as no noticeable change in user perception and/or safety) of at least 4 months, at least 5 months, at least 6 months, at least 7 months, or at least 8 months.

In some examples, the smokeless article may have a mass of about 0.1 g to 5.0 g. In some examples the smokeless article has a mass of about 0.5 g to about 4.0 g. In some examples the smokeless article has a mass of about 1.0 g to about 3.0 g.

In some examples, the smokeless article has a length of about 30 mm. In some examples the smokeless article has a length of about 28 mm. In some examples, the smokeless article has a length of about 26 mm. In some examples the smokeless article has a width of about 12mm. In some examples the smokeless article has a width of about 10 mm. In some examples the smokeless article has a width of about 8 mm. In some examples the smokeless article has a depth of about 5 mm. In some examples the smokeless article has a depth of about 4 mm. In some examples the smokeless article has a depth of about 3 mm.

In some examples, the smokeless article may have an active lifetime of about 20 minutes to about 60 minutes after being placed in the mouth. In some examples, the smokeless article has an active lifetime of about 25 minutes to 50 minutes after being placed in the mouth. In some examples, the smokeless article has an active lifetime of about 30 minutes to about 45 minutes after being placed in the mouth. As used herein, the term "active lifetime" is intended to refer to the amount of time after being placed in the mouth that the smokeless article provides the user with a perceptible taste and/or physiological experience. For example, the smokeless articles described herein comprises a nicotinic component - the active lifetime may be defined as the in use period of time within which 90 wt% of the available nicotine is released. In other words, the active lifetime may be the duration of time from insertion into the oral cavity for 90 wt% of the total amount of nicotine that is capable of being released during normal use to dissolve into the user's saliva and/or enter the user's bloodstream. It will therefore be appreciated that the active lifetime of a product may vary from user to user and for a user based on oral conditions, in particular extent of salivation. Nonetheless, the skilled person is able to mimic oral conditions to determine the active lifetime in one instance, which can be used as a comparison or analysis point.

In some examples the content which comprises the nicotinic component comprises less than 5 wt% tobacco. "Tobacco" as used herein refers to tobacco, or any tobacco derived product excluding nicotinic components.

In some examples the first and/or second content is free of tobacco. In this way, the user may experience a similar or enhanced recreational/pharmaceutical effect as compared to conventional tobacco-containing products without experiencing undesirable components inherent to tobacco (e.g. tobacco flavour).

In some examples of the smokeless article, the first content comprises the nicotinic component and the second content does not comprise the nicotinic component.

In such examples, the first content, in particular the pH of the first component, can be tuned to improve the release, absorption and perception of the nicotinic component by a user. At the same time, the second content does not comprise the nicotinic component. The second content, in particular the pH of the second content, can be tuned to improve the release, absorption and perception of some other component of the second content that is not nicotine and that benefits from being formulated in different conditions (i.e. different pH) to the nicotinic component.

In some examples of the smokeless article, the pH of the first content is in the range 7.5 to 10. In some examples of the smokeless article, the pH of the first content is in the range 7.5 to 9.5. In some examples of the smokeless article, the pH of the first content is in the range 8.4 to 9.

In some examples, the pH of the first content is basic. In some examples, the pH of the first content is ≥ 7.5, ≥ 7.75, ≥ 8, ≥ 8.25, or ≥ 8.4. In some examples, the pH of the first content is ≤ 10, ≤ 9.5, ≤ 9.25, or ≤ 9. The foregoing pH values may be combined in any way to generate a range, for example, the pH of the first content may be ≥ 7.75 and ≤ 9.25, of may be ≥ 7.5 and ≤ 8.

In some examples, the pH of the first content is about 8.4, about 8.5, about 8.6, about 8.7, about 8.8, about 8.9, or about 9. The foregoing pH values may be combined in any way to generate a range, for example, the pH of the first content may be ≥ 8.4 and ≤ 8.9.

In some examples of the smokeless article, the pH of the second content is in the range 4 to 7.5. In some examples of the smokeless article, the pH of the second content is in the range 6 to 7.5.

In some examples, the pH of the second content is more acidic than the pH of the first content. In some examples, the pH of the second content is approximately neutral, or is acidic.

In some examples, the pH of the second content is ≥ 4, ≥ 5, ≥ 6, ≥ 6.25 or ≥ 6.5. In some examples, the pH of the second content is ≤ 7.5, ≤ 7.25, or ≤ 7. The foregoing pH values may be combined in any way to generate a range, for example, the pH of the second content may be ≥ 6.25 and ≤ 7.25, or may be ≥ 6 and ≤ 6.5.

In some examples, the pH of the second content is about 4, about 4.1, about 4.2, about 4.3, about 4.4, about 4.5, about 4.6, about 4.7, about 4.8, about 4.9, about 5, about 5.1, about 5.2, about 5.3, about 5.4, about 5.5, about 5.6, about 5.7, about 5.8, about 5.9, about 6, about 6.1, about 6.2, about 6.3, about 6.4, about 6.5, about 6.6, about 6.7, about 6.8, about 6.9, about 7, about 7.1, about 7.2, about 7.3, about 7.4, or about 7.5. The foregoing pH values may be combined in any way to generate a range, for example, the pH of the first content may be ≥ 6.6 and ≤ 7.2.

In some examples of the smokeless article, the pH of the first content and the pH of the second content are different by at least 0.5 pH units.

In some examples, the pH of the first content and the pH of the second content are different by at least 0.1, at least 0.2, at least 0.3, at least 0.4, at least 0.5, at least 0.6, at least 0.7, at least 0.8, at least 0.9, at least 1, at least 1.1, at least 1.2, at least 1.3, at least 1.4, at least 1.5, at least 1.6, at least 1.7, at least 1.8, at least 1.9, at least 2, at least 2.1, at least 2.2, at least 2.3, at least 2.4, or at least 2.5 pH units.

In some examples of the smokeless article, the first and/or second content each independently contain a pH buffer.

In some examples, the pH buffer is at least partially responsible for setting, maintaining or altering the pH of the first and/or second contents.

The first and/or second content each independently contain a pH buffer - this means that the pH buffer in the first content may be the same or different to the pH buffer in the second content.

As used herein, the terms "pH regulator", "buffer", "pH stabiliser", "pH modifier", and "pH adjuster" interchangeably refer to components of the first and/or second content used to control pH. Within these definitions, the terms "pH regulator", "buffer" and "pH stabiliser" refer to compounds that have the capacity to resist pH change to some level so providing some ability to maintain pH in a specific buffer range as compared to just altering the pH.

Examples of suitable pH buffers include ammonia, ammonium carbonate, sodium carbonate, calcium lactate, and calcium carbonate. These components may have a buffering action, which helps to maintain the pH at the desired level.

The term "carbonate" may be used to refer to bicarbonate, carbonate, and mixtures thereof. In some examples, "carbonate" is carbonate. In some examples "carbonate" is bicarbonate. In some examples, "carbonate" is a mixture of carbonate and bicarbonate.

In some examples of the smokeless article, the second content comprises a pH sensitive component that is incompatible with the pH of the first content, or the first content comprises a pH sensitive component that is incompatible with the pH of the second content.

A pH sensitive component is any chemical moiety that is responsive in any way to the pH of its environment. pH might affect the chemical state / nature, the physical state / nature / appearance, the performance, the user perception, the solubility, the stability, the reactivity or something else of the pH sensitive component.

In some examples, the pH sensitive component is incompatible with the pH of the content that it is not contained in, e.g. if contained in the first content, the pH sensitive component is incompatible with the pH of the second content, and vice versa. Here, by incompatible it is meant that the pH sensitive component is in some way not suited to a given pH. It may be that the incompatibility manifests in undesirable instability, appearance, solubility, reactivity, user perception, or some other undesirable characteristic. It may be that the incompatibility results in the pH sensitive component generally performing sub-optimally as a result of the pH of its environment.

In some examples of the smokeless article, the second content comprises a pH sensitive component that is compatible with the pH of the second content, and/or the first content comprises a pH sensitive component that is compatible with the pH of the first content.

In some examples the pH sensitive component is compatible with the pH of the content that it is contained in. Here, by compatible it is meant that the pH sensitive component is in some way suited to a given pH. It may be that the compatibility manifests in desirable stability, appearance, solubility, reactivity, user perception, or some other desirable characteristic. It may be that the compatibility results in the pH sensitive component generally performing optimally, close to optimally, or more optimally as a result of the pH of its environment.

The present invention beneficially allows a pH sensitive component, that is otherwise not compatible with the pH of either the first content or the second component, to be more optimally used in a smokeless article that includes said first or second component with which the pH sensitive component is not compatible. More broadly, simultaneous formulation of pH sensitive components / contents is made possible in the present invention.

In some examples of the smokeless article, the second content comprises a pH sensitive component that is incompatible with the pH of the first content.

In some examples, the pH sensitive component is specifically incompatible with the pH of the first content which comprises a nicotinic component. In such examples, the nicotinic component can be formulated in the first compartment for optimal performance (i.e. solubility, absorption and user perception), whilst the pH sensitive component is formulated in the second content, with a different pH to the first content, where it performs more optimally than if it were in the first content comprising the nicotinic component.

In some examples of the smokeless article, the pH sensitive component comprises one or more of a flavouring, a salt, a humectant, an active ingredient, a filler, a sweetener, a stabiliser and a binder.

In some examples of the smokeless article, the pH sensitive component comprises a flavouring. In some examples, the pH sensitive component comprises vanillin, ethyl vanillin, citric acid, or a combination thereof. In some examples, the pH sensitive component is an acidic component.

In some examples of the smokeless article, the pH sensitive component comprises the nicotinic component, the pH sensitivity relating to detrimental release or absorption profile under certain pH conditions.

In some examples, the first content comprises a flavouring. Additionally or alternatively, in some examples, the second content comprises a flavouring. Flavouring may be provided in solid or liquid form. Suitable flavourings include coffee, eucalyptus, menthol, liquorice, peppermint, spearmint, chocolate, fruit flavour (including e.g. citrus, cherry etc.), vanilla, spice (e.g. ginger, cinnamon) and tobacco flavour. The flavouring may be evenly dispersed throughout the content in which it is contained or may be provided in isolated locations and/or varying concentrations throughout the content in which it is contained. As used herein, the term "flavouring" denotes a compound having a desirable taste, aroma or both. In some examples, the flavouring is vanilla (i.e. comprises vanillin).

In some examples, the content containing the flavouring comprises one or more flavourings (e.g. peppermint flavour) in an amount of from above 0 wt.% to 10 wt.%, from 1 wt.% to 10 wt.%, from 1 wt.% to 8 wt.%, from 1 wt.% to 6 wt.%, from 2 wt.% to 8 wt.%, from 2 wt.% to 7 wt.%, or from 3 wt.% to 7 wt.% based on the total weight of the content.

In some smokeless articles flavouring materials included in a content may cause the content to become more acidic. In cases where the flavouring materials have a greater impact on the pH of the content the use of a combination of pH regulators selected from the list of sodium carbonate, sodium hydroxide, calcium lactate, sodium phosphate dibasic, sodium citrate, meglumine, ammonia, ammonium carbonate, and calcium carbonate may be particularly useful to control the pH to within a desired range.

When the pH sensitive component is a flavouring, it may be that if the flavouring were formulated at a pH with which it is incompatible, discolouration or another kind of spoiling occurs. For example, vanillin discolours in a basic environment.

In some examples, the pH sensitive component comprises a salt. In some examples, the first content comprises a salt. In some examples the second content comprises a salt.

In some examples of the smokeless article, the salts may be provided to maintain the osmolarity of the content within the smokeless article. Salts contribute to the overall positive user experience of the smokeless article, such as pleasant mouth feel. Examples of salts which may be used include sodium chloride, potassium chloride, magnesium chloride and calcium chloride.

In some examples of the smokeless article, the pH sensitive component comprises a humectant. In some examples, the first content comprises a humectant. In some examples, the second content comprises a humectant.

Humectants may be provided to control moisture content thereby preventing the smokeless article from drying out during storage and reducing the amount of saliva wetting required before the user experience begins. Suitable humectants include polyhydric alcohols (e.g. propylene glycol (PG), triethylene glycol, 1 ,2-butane diol and glycerol such as vegetable glycerine (VG)) and their esters (e.g. glycerol mono-, di- or tri-acetate). The humectant component may consist of a single humectant compound or may consist of two or more different humectant compounds.

In some examples, the pH sensitive component comprises an active ingredient. In some examples, the first content comprises an active ingredient. In some examples, the second content comprises an active ingredient. In some examples, the active ingredient is a biologically and/or pharmaceutically active ingredient.

Active ingredients (i.e. biologically/pharmacologically active compounds) are provided to produce a pharmacological effect in the user. Suitable active compounds include the group consisting of: nicotine, cannabinoids, caffeine, opiates and opioids, cathine and cathinone, kavalactones, mysticin, beta-carboline alkaloids, salvinorin A, together with any combinations, functional equivalents to, and/or synthetic alternatives of the foregoing. Active ingredients (i.e. biologically/pharmacologically active compounds) may also have additive properties. In some examples, active ingredients are present at 1-3 wt% relative to the total weight of the content.

In some examples the pH sensitive component comprises a filler. In some examples the first content comprises a filler. In some examples the second content comprises a filler.

In some examples, the filler comprises one or more natural fibres. In some examples the natural fibres comprise one or more of micro-crystalline cellulose, wheat fibre, bamboo fibre, apple fibre, citrus fibre, sugar cane fibre, rice fibre, psyllium fibre, potato fibre, pea fibre, and oat fibre.

Fillers may be provided to increase the volume of the smokeless article and the volume of the first and/or second compartments (e.g. by increasing the volume contained within the smokeless article and to strengthen the first and/or second contents).

In general, suitable fillers include calcium carbonate, calcium phosphate, corn starch, grains, lactose, polysaccharides (e.g. maltodextrin), polyols, sugars (e.g. dextrose, mannitol, xylitol, sorbitol), natural fibres (e.g. non-tobacco fibres), microcrystalline cellulose, cellulose and cellulose derivatives (e.g. finely divided cellulose), lignocellulose fibres (e.g. wood fibres), jute fibres and combinations thereof. In some cases the filler comprises wheat fibres. In some cases the filler comprises bamboo fibres. The use of a filler improves the overall mouth-feel of the smokeless article.

In some examples the filler comprises micro-crystalline cellulose and one of wheat fibres or bamboo fibres.

In some cases, the filler content is about 30 to 70 wt% of the content. In some examples, the filler content is about 40 to 60 wt% of the content.

In some examples, the pH sensitive component comprises plant material. In some examples the first content comprises plant material. In some examples, the second content comprises plant material.

In some examples, the plant material functions as a filler.

Plant material may be provided for physical integrity and may function as a natural source of substances such as, for example, biologically/pharmacologically active compounds, flavourings, pH stabilisers etc. The plant material may comprise at least one plant material selected from the list including *Amaranthus dubius*, *Arctostaphylos uva-ursi* (Bearberry), *Argemone mexicana*, *Amica*, *Artemisia vulgaris*, Yellow Tees, *Galea zacatechichi*, *Canavalia maritima* (Baybean), *Cecropia mexicana* (Guamura), *Cestrum noctumum*, *Cynoglossum virginianum* (wild comfrey), *Cytisus scoparius*, *Damiana*, *Entada rheedii*, *Eschscholzia califomica* (California Poppy), *Fittonia albivenis*, *Hippobroma longiflora*, *Humulus japonica* (Japanese Hops), *Humulus lupulus* (Hops), *Lactuca virosa* (Lettuce Opium), *Laggera alata*, *Leonotis leonurus*, *Leonurus cardiaca* (Motherwort), *Leonurus sibiricus* (Honeyweed), *Lobelia cardinalis*, *Lobelia inflata* (Indian-tobacco), *Lobelia siphilitica*, *Nepeta cataria* (Catnip), *Nicotiana species* (Tobacco), *Nymphaea alba* (White Lily), *Nymphaea caerulea* (Blue Lily), Opium poppy, *Passiflora incamata* (Passionflower), *Pedicularis densiflora* (Indian Warrior), *Pedicularis groenlandica* (Elephant's Head), *Salvia divinorum*, *Salvia dorrii* (Tobacco Sage), Salvia species (Sage), *Scutellaria galericulata*, *Scutellaria lateriflora*, *Scutellaria nana*, *Scutellaria* species (Skullcap), *Sida acuta* (Wireweed), *Sida rhombifolia*, *Silene capensis*, *Syzygium aromaticum* (Clove), *Tagetes lucida* (Mexican Tarragon), *Tarchonanthus camphoratus*, *Tumera diffusa* (Damiana), *Verbascum* (Mullein), *Zamia latifolia* (Maconha Brava) together with any combinations, functional equivalents to, and/or synthetic alternatives of the foregoing.

In some examples, the filler may have a particle size of 10-500 µm, 10-450 µm, 10-400 µm, 10-350 µm, 10-300 µm, 10-250 µm, 10-200 µm, 10-150 µm, 10-100 µm, 10-50 µm, 15-50 µm, 10-450 µm, 15-45 µm, 20-50 µm, 20-45 µm, 20-40 µm, or 25-35 µm.

In some examples, the filler may have a particle size of 15-500 µm, 20-500 µm, 50-500 µm, 100-500 µm, 150-500 µm, 200-500 µm, 250-500 µm, 250-450 µm, 250-400 µm, 250-350 µm, 260-340 µm, 270-330 µm, 280-320 µm, 290-310 µm, or 295-305 µm.

As used herein, the term "particle size" when referring to fibres may indicate the maximum size of the longest dimension of the fibres. For example, a particle size of 50 µm indicates that in the population of fibres, the maximum fibre length is 50 µm. Alternatively, the term "particle size" when referring to fibres may indicate the average size of the longest dimension of the fibres.

Particles having the desired particle size may be obtained by passing a population of fibres through a sieve of corresponding mesh size. For example, to obtain fibres of particle size 300 µm (i.e. a maximum fibre length of 300 µm), a population of fibres are passed through a sieve with 300 µm diameter apertures in the mesh. In this way, fibres with a length of 300 µm or less pass through the mesh and fibres longer than 300 µm are retained by the mesh. The fibres which pass through the mesh may then be used in the smokeless article of the invention, having a particle size of 300 µm. Fibres of a desired particle size are also available from commercial suppliers such as Jelu-werk

In some examples, the pH sensitive component comprises a binder. In some examples, the first content comprises a binder. In some examples, the second content comprises a binder.

Suitable binders include starches and/or cellulosic binders such as methyl cellulose, ethyl cellulose, hydroxypropyl cellulose, hydroxyethyl cellulose and carboxymethyl cellulose, gums such as xanthan, guar, arabic and/or locust bean gum, organic acids and their salts such as alginic acid (sodium alginate), agar and pectins.

In some examples of the smokeless article, the pH sensitive component comprises a sweetener. In some examples, the first content comprises a sweetener. In some examples, the second content comprises a sweetener.

Sweeteners may be provided to modify the user taste perception and, in particular, overcome bitter flavours that result from other substances. Suitable sweeteners include honey, sugar, brown sugar, glucose, fructose, sucrose, aspartame, xylitol, maltitol, saccharin sodium, glycyrrhizin tripotassium liquorice, jujube, acesulfame K, sucralose or a mixture thereof. In some cases the sweetener may be xylitol.

In some examples, the first and/or second content comprises a sweetener in an amount of from 0 wt.% to 5 wt.%, from 0.01 wt.% to 4 wt.%, from 0.01 wt.% to 3 wt.%, from 0.5 wt.% to 3 wt.%, or from 1 wt.% to 3 wt.% based on the total weight of the first and/or second content.

In some examples of the smokeless article, the pH sensitive component comprises a stabiliser. In some examples, the first content comprises a stabiliser. In some examples the second content comprises a stabiliser.

Stabilisers are provided to prevent decomposition or degradation over time during storage by, for example, retarding oxidation or unwanted biological activity. Stabilisers may be selected from the group consisting of antioxidants including vitamin E, such as tocopherole, ascorbic acid, sodium pyrosulfite, butylhydroxytoluene, butylated hydroxyanisole, edetic acid and salts thereof; and preservatives including citric acid, tartaric acid, lactic acid, malic acid, acetic acid, benzoic acid, sorbic acid and salts thereof.

In some examples of the smokeless article, the barrier comprises a weld between two or more surfaces of the water-permeable layer.

In some examples, the weld is a heat held, where two or more surfaces of the water-permeable layer (e.g. the water-permeable layer defining the outer surface of a smokeless article) are fused together. In some examples heat welding is carried out using heated anvils.

In some examples, the barrier comprises a layer of material, for example a water permeable material as described herein, that prevents physical passage of the first and second contents between first and second compartments. In some examples, the barrier is water-permeable. In some examples, the barrier is not water permeable. In some examples, the barrier comprises a gel, for example a gel plug. In some examples, the gel comprises gelatine, agar, hydrogel, or mixtures thereof. In some examples, the gel (e.g. gel plug) is water permeable.

In some examples, depending on the shape of the smokeless article and the first and second compartments, the barrier might be formed from more than one barrier portion, for example more than one weld, or more than one layer of material.

In a second aspect the present disclosure provides a method of manufacturing the smokeless article described herein, comprising: laser cutting of the material used to form the water permeable layer to form shaped elements for forming the smokeless article; and thermoforming to seal the shaped elements to form the smokeless article.

In some examples, the method of manufacturing the smokeless article comprises thermoforming to partially seal the shaped elements. In such examples, filling of the first and second compartments takes place to fill the partially formed compartments. In some examples, the first and second content is present before thermoforming and sealing is complete.

In some examples the shape of the first compartment is the same as the second compartment. In some examples the shape of the first compartment is different to the second compartment. In some examples the volume of the first compartment is the same as the volume of the second compartment. In some examples the volume of the first compartment is different to the volume of the second compartment.

In some examples the first and/or second compartment has a regular or irregular polygonal shape. In some examples the first and/or second compartment has a triangular shape. In some examples the first and/or second compartment has a square shape. In some examples the first and/or second compartment has a pentagonal shape. In some examples the first and/or second compartment has a hexagonal shape. In some examples the first and/or second compartment has a heptagonal shape. In some examples the first and/or second compartment has an octagonal shape. In some examples the first and/or second compartment has a trapezoidal shape. In some examples the first and/or second compartment is shaped to be a segment of a circle. In some examples the first and/or second compartment is shaped as a whole, or a portion of a circle or an oval shape. In some examples the first and/or second compartment have a graphical shape. That is, in some examples, the shape of the compartment depicts an image (for example, the shape of an apple, or a chilli pepper). In some examples, one or more corners present on the smokeless article are rounded. In some examples, "shape" refers to shape in a lateral plane of the smokeless article.

In some examples, the first compartment has a greater volume than the second compartment. In some examples, the first compartment has a volume that is 50%, 100%, 200%, 300%, 400% or 500% greater than the second compartment. The second compartment being small / smaller compared to the first compartment allows, in some examples, the first content, which may include a nicotinic component, to be present in larger quantities than the second content, which for example, may contain a flavouring that is incompatible with the pH of the first content. Accordingly, in some examples, there is 50% more, 100% more, 200% more, 300% more, 400% more, or 500% more first content than second content in the smokeless article.

In some examples, the smokeless article is longitudinally symmetric. In such examples, the production of the smokeless article can be made easier and simpler. In some examples, the smokeless article has a rectangular shape and is longitudinally symmetric. In such examples, different volumes of the first and second compartments can be achieved by having a non-central barrier, that is closer to one of the edges of the rectangular-shaped smokeless article than the other, equivalent, edge (e.g. closer to one short edge than the other short edge). In such examples the barrier may be described as "offset" or "centrally offset".

In some examples, the barrier defines an edge or a face of a compartment. In some examples, the first and second compartment are side-by-side in the smokeless article. In such examples it may be that the barrier defines an edge to the compartment. In some examples, the first and second compartment are on top of one another. In such examples it may be that the barrier defines a face of the compartment.

In some examples each of the first and second compartments includes a portion of the compartment wall which defines the outer wall of the smokeless article. In this case, neither first nor second compartment is completely formed within the other with no wall that is part of the outer surface of the smokeless article. In this way, saliva from a user can penetrate both first and second compartments directly, without passing through the other compartment.

In some examples, the colours of the first and second compartment are different. In some examples, the smokeless article and/or the first and second compartments may be coloured or include markings, such as brand logos and text, to improve user perception. The smokeless article may be partially or completely coloured by a colourant.

Colourants may be provided to modify the user impression of the smokeless article. Colourants include whitening agents. Colourants may be selected from one or more of common colourants such as curcumin (E100), turmeric (E100(ii)), riboflavin (E101), riboflavin-5'-phosphate (E101(ii)), tartrazine (E102), quinoline yellow (E104), riboflavin-5-sodium phosphate (E106), yellow 2G (E107), sunset yellow FCF (E110), carmine, cochineal (E120), azorubine (E122), amaranth (E123), ponceau 4R (E124), erythrosine (E127), red 2G (E128), allura red AC (E129), patent blue V (E131), indigotine (E132), brilliant blue FCF (E133) chlorophylls (E140), copper complexes of chlorophyll (E141), green S (E142), caramel (E150a-d), brilliant black BN (E151), carbon (E153), brown FK (E154), brown HT (E155), alfa-, beta- and gamma- carotene (E160a), annatto, bixin, norbixin (E160b), bell pepper (Paprika) extract (E160c), lycopene (E160d), beta- apo-8'-carotenal (E160e), ethyl ester of beta-apo-8'-carotenic acid (E160f), flavoxanthin (E161a), lutein (E161b), cryptoxanthin (E161c), rubixanthin (E161d), violaxanthin (E161e), rhodoxanthin (E161f), canthaxanthin (E1619), citranaxanthin (E161h), beetroot extract (E162), anthocyanins (E163), calcium carbonate (E170), titanium dioxide (E171), iron oxides (E172), aluminium (E173), silver (E174), gold (E175), lithol rubine BK (E180), tannins (E181). The amount of colourant may be up to about 3% by weight of the smokeless article, such as about 0.5% to about 2.5% or about 1% to about 2%.

In some examples, the smokeless article comprises a third compartment. In some examples the smokeless article comprises a fourth compartment. In some examples, when the smokeless article comprises a third and/or third and fourth compartment, there may be more than one barrier, and each barrier may comprise more than one barrier portion (i.e. more than one weld or layer of material).

In some examples, there are 1, 2, 3 or 4 barriers. Where there are multiple barriers, the barrier may be formed in the same, or a different way (for example, one barrier may be a weld, the other a separate layer of material).

If present, in some examples the third and fourth compartments are defined in the same way as the first and second compartments are defined above.

Where more than two compartments are present, each compartment may contain mutually incompatible components that are physically separated from each other by the barriers.

In some examples, the third and fourth compartments comprise third and fourth contents, respectively, that can be the same or different to the first and second contents. In some examples, the pH of the third and/or fourth compartments (when present) is different to the pH of one or more (for example, all) the other compartments present in the smokeless article.

In a third aspect, the present disclosure provides a kit comprising a plurality of smokeless articles according to the first aspect. In some examples the kit comprises 2 to 20 smokeless articles.

In a fourth aspect, the present disclosure provides the use of a smokeless article as described herein to more optimally formulate a pH sensitive component in a smokeless article.

The preceding summary is provided for purposes of summarizing some examples to provide a basic understanding of aspects of the subject matter described herein. Accordingly, the above-described features should not be construed to narrow the scope or spirit of the subject matter described herein in any way. Moreover, the above and/or proceeding examples may be combined in any suitable combination to provide further examples, except where such a combination is clearly impermissible or expressly avoided. Other features, aspects, and advantages of the subject matter described herein will become apparent from the following text and the accompanying drawings.

### BRIEF DESCRIPTION OF THE FIGURES

Aspects, features and advantages of the present disclosure will become apparent from the following description of examples in reference to the appended drawings in which like numerals denote like elements.
**Fig. 1** shows a cross sectional view of four embodiments (a) - d)) of a smokeless article.
**Fig. 2** shows a cross sectional view of four embodiments (e) - h)) of a smokeless article.
**Fig. 3** shows a bar chart of L*a*b* measurements over a period of nine months for a smokeless article that is outside of the scope of the invention described herein and which includes vanillin as a flavouring.
**Fig. 4** shows a bar chart of L*a*b* measurements over a period of nine months for a smokeless article that is outside of the scope of the invention described herein and which includes a berry flavouring.
**Fig. 5** shows a bar chart of ΔL, Δa and Δb measurements over a period of nine months for a smokeless article that is outside of the scope of the invention described herein and which includes vanillin as a flavouring.
**Fig. 6** shows a bar chart of ΔL, Δa and Δb measurements over a period of nine months for a smokeless article that is outside of the scope of the invention described herein and which includes a berry flavouring.

### DETAILED DESCRIPTION OF EMBODIMENTS

Before describing several examples implementing the present disclosure, it is to be understood that the present disclosure is not limited by specific construction details or process steps set forth in the following description and accompanying drawings. Rather, it will be apparent to those skilled in the art having the benefit of the present disclosure that the systems, apparatuses and/or methods described herein could be embodied differently and/or be practiced or carried out in various alternative ways.

Unless otherwise defined herein, scientific and technical terms used in connection with the presently disclosed inventive concept(s) shall have the meanings that are commonly understood by those of ordinary skill in the art, and known techniques and procedures may be performed according to conventional methods well known in the art and as described in various general and more specific references that may be cited and discussed in the present specification.

Any patents, published patent applications, and non-patent publications mentioned in the specification are hereby incorporated by reference in their entirety.

All examples implementing the present disclosure can be made and executed without undue experimentation in light of the present disclosure. While particular examples have been described, it will be apparent to those of skill in the art that variations may be applied to the systems, apparatus, and/or methods and in the steps or in the sequence of steps of the methods described herein without departing from the concept, spirit, and scope of the inventive concept(s). All such similar substitutions and modifications apparent to those skilled in the art are deemed to be within the spirit, scope, and concept of the inventive concept(s) as defined by the appended claims.

The use of the term "a" or "an" in the claims and/or the specification may mean "one," as well as "one or more," "at least one," and "one or more than one." As such, the terms "a," "an," and "the," as well as all singular terms, include plural referents unless the context clearly indicates otherwise. Likewise, plural terms shall include the singular unless otherwise required by context.

The use of the term "or" in the present disclosure (including the claims) is used to mean an inclusive "and/or" unless explicitly indicated to refer to alternatives only or unless the alternatives are mutually exclusive. For example, a condition "A or B" is satisfied by any of the following: A is true (or present) and B is false (or not present), A is false (or not present) and B is true (or present), and both A and B are true (or present).

As used in this specification and claim(s), the words "comprising, "having," "including," or "containing" (and any forms thereof, such as "comprise" and "comprises," "have" and "has," "includes" and "include," or "contains" and "contain," respectively) are inclusive or open-ended and do not exclude additional, unrecited elements or method steps.

Unless otherwise explicitly stated as incompatible, or the physics or otherwise of the embodiments, examples, or claims prevent such a combination, the features of examples disclosed herein, and of the claims, may be integrated together in any suitable arrangement, especially ones where there is a beneficial effect in doing so. This is not limited to only any specified benefit, and instead may arise from an "ex post facto" benefit. This is to say that the combination of features is not limited by the described forms, particularly the form (e.g. numbering) of example(s), embodiment(s), or dependency of claim(s). Moreover, this also applies to the phrase "in one embodiment," "according to an embodiment," and the like, which are merely a stylistic form of wording and are not to be construed as limiting the following features to a separate embodiment to all other instances of the same or similar wording. This is to say, a reference to 'an,' 'one,' or 'some' embodiment(s) may be a reference to any one or more, and/or all embodiments, or combination(s) thereof, disclosed. Also, similarly, the reference to "the" embodiment may not be limited to the immediately preceding embodiment. Further, all references to one or more embodiments or examples are to be construed as non-limiting to the claims.

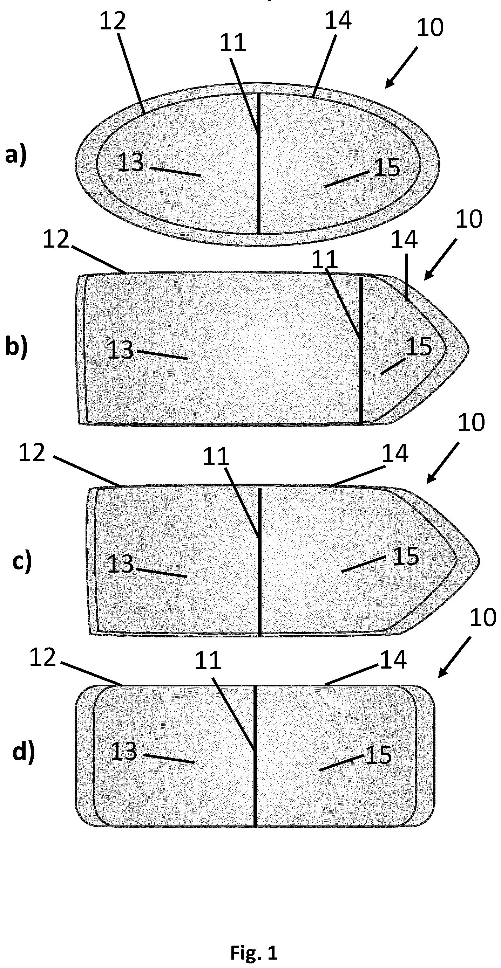

As shown in Fig. 1, there is schematically provided four embodiments of the smokeless article 10 of the present invention. The embodiments comprise a first compartment 12 and a second compartment 14. The first compartments 12 are substantially filled with first contents 13. The second compartments 14 are substantially filled with second contents 15. The first compartment 12 and the second compartment 14 are separated by a barrier 11, such that the first content 13 and the second content 15 cannot move between the first compartment 12 and the second compartment 14.

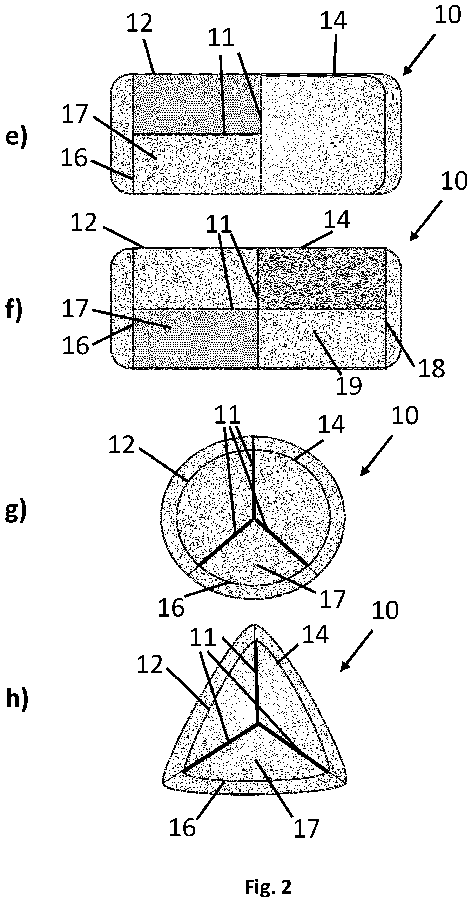

In Fig. 1 a) the first 12 and second 14 compartments are of a semi-oval shape and are approximately the same size. In Fig. 2 b) the first compartment 12 is rectangular in shape, and the second compartment 14 is triangular in shape, and the first compartment 12 is much larger than the second compartment 14. In Fig. 1 c) the first compartment 12 is rectangular in shape and the second compartment 14 is trapezoidal in shape, and the second compartment 14 is slightly larger than the first compartment 12. In Fig. 1 d) the first compartment 12 and the second compartment 14 are rectangular in shape and are approximately the same size.

Fig. 2 schematically provides embodiments of the smokeless article 10 of the present invention, where there are more than two compartments. Fig. 2 e), g) and h) each illustrate a smokeless article 10 that comprises a third compartment 16. Fig. 2 f) comprises a third compartment 16 and a fourth compartment 18. The third compartment 16 encompasses a third content 17, and the fourth compartment 18 encompasses a fourth content 19. In Fig. 2, the smokeless articles comprise more than one barrier 11 / more than one barrier portion separating the various compartments. In Fig. 2, all embodiments include first contents 13 in first compartments 12, and second contents 15 in second compartments 14, but this is not shown.

In each embodiment illustrated in Fig. 1 and Fig. 2, the first content has a different pH to the second content, and at least one of the first and second content comprises a nicotinic component.

### Example 1

The inventors have found that vanillin, which is a component of vanilla flavours, discolours in basic environments (see comparative Example 1). It is often preferable to formulate a smokeless article to have a content that creates a basic environment (for example, pH 8.4 - 9.0) as this can improve nicotine absorption by a user, and in turn improve the overall perception of the smokeless article by a user. The inventors have found that vanillin discolours in said basic environment which is formulated to have a pH that promotes the performance of nicotine. As such, vanillin is incompatible with the pH of such a content.

The present invention allows vanillin to be formulated in a separate content, with a different pH to the content that includes the nicotinic component. In particular, the vanillin can be formulated in a content with a pH of 6 - 7.5, where no discolouration occurs. In this way, vanillin (a pH sensitive component) can be more optimally formulated in the same smokeless article as a nicotinic component, despite being incompatible with the pH of the content which includes the nicotinic component.

Provided below is a generic formulation of a smokeless article according to the present invention. A range of possible wt.% values are given for each component. According to the present invention, the first content in the first compartment includes nicotine and has a more basic pH to improve nicotine absorption by a user. The second content in the second compartment does not include nicotine and so does not require said basic pH for optimal nicotine delivery. The second content includes a pH-sensitive component that is a flavour mix, and is formulated to be at a different pH to the first content (for example, pH 6 - 7.5 in the case of vanillin). Each of the first and second content contain a buffer, for maintaining a desired pH level.

### First content in first compartment

| Material | Approx wt.% values | |
|---|---|---|
| | Lower range | Upper range |
| Nicotine | 0.2 | 1.6 |
| Solvent | 35 | 50 |
| Bulking Agent 1 | 15 | 32 |
| Bulking Agent 2 | 0 | 25 |
| Bulking Agent 3 | 0 | 15 |
| Humectant 1 | 4 | 10 |
| Humectant 2 | 0 | 3 |
| Buffering Agent | 0.1 | 0.6 |

### Second content in second compartment

| Material | Approx wt.% values | |
|---|---|---|
| | Lower range | Upper range |
| Solvent | 7 | 50 |
| Bulking Agent 1 | 15 | 32 |
| Bulking Agent 2 | 0 | 25 |
| Bulking Agent 3 | 0 | 15 |
| Humectant 1 | 4 | 10 |
| Humectant 2 | 0 | 3 |
| Buffering Agent | 0 | 0.6 |
| Flavour mix | 2 | 14 |

### Comparative Example 1

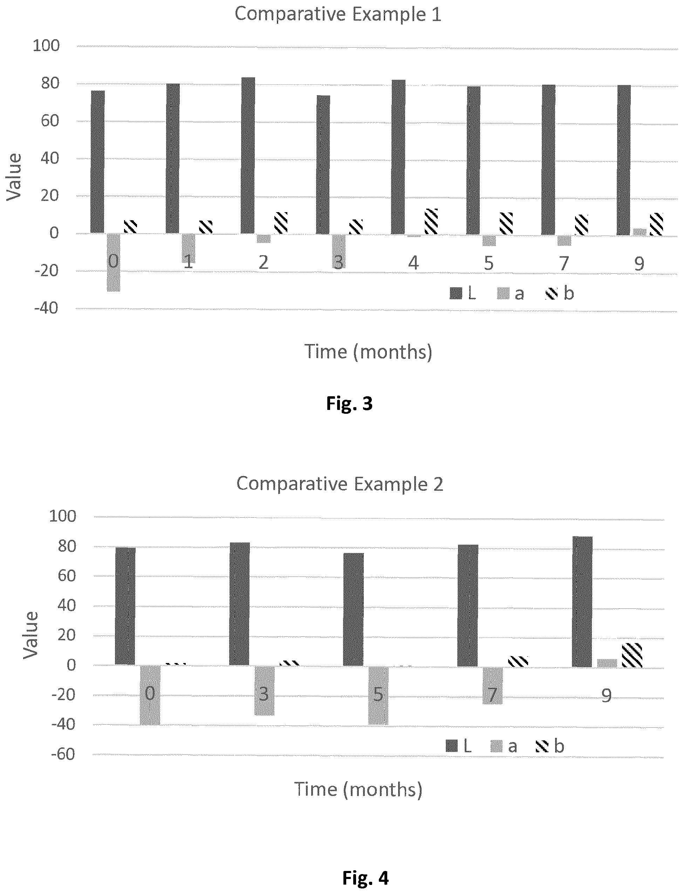

Fig. 3 is a bar chart visually representing the L*a*b* data in Table 1a below. Comparative Example 1 makes use of single compartment smokeless articles which includes vanillin flavouring in the same content as the nicotinic component. The content in Comparative Example 1 has a relatively basic pH, at t=0 measured as 8.11. The L value represents lightness, a more positive value being lighter and a more negative value being darker. The a value represents red/green, a more positive value being more red, and a more negative value being more green. The b value represents yellow/blue, a more positive value being more yellow and a more negative value being more blue. The pouches tested to generate the data in Fig. 3 exhibit a rapid and significant change in the a value (i.e. a green-red shift), which is clear even after one month. The effect of this is an overall discolouration with a yellow colour developing overtime. It is evident that the vanillin flavouring is incompatible with the pH of the content that includes a nicotinic component.

**Table 1a: L*a*b* data for smokeless articles featuring a single compartment comprising a content that includes vanillin flavouring (liquid) and a nicotinic component. Smokeless articles are stored at room temperature.**

| **Colourimetry** | **Condition - Ambient** | | | |
|---|---|---|---|---|
| | **Time (months)** | **L** | **a** | **b** |
| **03/10/2023** | 0 | 76.05 | -30.93 | 7.18 |
| **02/11/2023** | 1 | 80.24 | -15.52 | 7.17 |
| **02/12/2023** | 2 | 83.58 | -4.44 | 11.95 |
| **01/01/2024** | 3 | 74.33 | -17.7 | 8.11 |
| **31/01/2024** | 4 | 82.93 | -1.13 | 14.16 |
| **01/03/2024** | 5 | 79.46 | -5.6 | 12.36 |
| **30/05/2024** | 7 | 80.67 | -5.29 | 11.44 |
| **26/07/2024** | 9 | 80.63 | 4.05 | 12.6 |

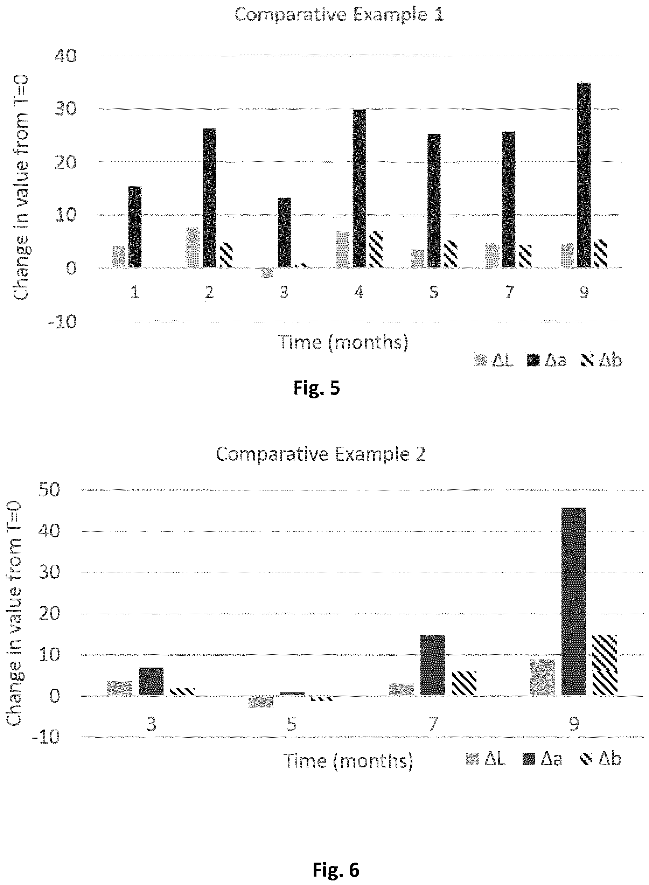

Fig. 5 is bar chart visually representing the ΔL, Δa and Δb data in Table 1b below. ΔL, Δa and Δb are calculated from the data in Table 1a, and for a given time point are calculated relative to the L, a and b values at time = 0 months. It is clear that there is a significant and immediate discolouration seen through the Δa values.

| ***Table 1b:** ΔL*, *Δa*, *and Δ*b *data generated from the L*a*b* data in Table 1a.* **Time (months)** | **ΔL** | **Δa** | **Δb** |
|---|---|---|---|
| **0** | 0.00 | 0.00 | 0.00 |
| **1** | 4.19 | 15.41 | -0.01 |
| **2** | 7.53 | 26.49 | 4.77 |
| **3** | -1.72 | 13.23 | 0.93 |
| **4** | 6.88 | 29.80 | 6.98 |
| **5** | 3.41 | 25.33 | 5.18 |
| **7** | 4.62 | 25.64 | 4.26 |
| **9** | 4.58 | 34.98 | 5.42 |

### Comparative Example 2

Fig. 4 is a bar chart visually representing the L*a*b* data in Table 2a below. Comparative Example 2 makes use of single compartment smokeless articles including berry flavouring in the same content as the nicotinic component. The berry flavouring does not contain vanillin. The content in Comparative Example 2 has a relatively basic pH, measured at t=0 as 8.64. The L value represents lightness, a more positive value being lighter and a more negative value being darker. The a value represents red/green, a more positive value being more red, and a more negative value being more green. The b value represents yellow/blue, a more positive value being more yellow and a more negative value being more blue. The pouches tested to generate the data in Fig. 4 exhibit significantly lower changes in L, a or b values, for example lower changes over a period of seven months. The development of a yellow colour over time is much less pronounced that in Comparative Example 1 (although some yellowing is still observed, particularly at later time points, for example at 9 months). Notably, unlike the pouches containing vanillin in Fig. 3, there is a significantly lower change in the a value for the majority of the test period (i.e. minimal green-red shift).

**Table 2a: L*a*b* data for smokeless articles featuring a single compartment comprising a content that includes berry flavouring (liquid) and a nicotinic component. Smokeless articles are stored at room temperature.**

| **Colourimetry** | **Condition - Ambient** | | | |
|---|---|---|---|---|
| | **Time (Months)** | **L** | **a** | **b** |
| **03/10/2023** | 0 | 79.18 | -39.85 | 1.73 |
| **01/01/2024** | 3 | 82.84 | -32.97 | 3.71 |
| **31/03/2024** | 5 | 76.26 | -38.94 | 0.59 |
| **30/05/2024** | 7 | 82.39 | -24.95 | 7.74 |
| **26/07/2024** | 9 | 88.16 | 5.91 | 16.56 |

Fig. 6 is bar chart visually representing the ΔL, Δa and Δb data in Table 2b below. ΔL, Δa and Δb are calculated from the data in Table 2a, and for a given time point are calculated relative to the L, a and b values at time = 0 months. There is no clear discolouration initially, and at least for the first five months. At 7 months Δa has increased, and at 9 months Δa has increased further, showing discolouration.

**Table 2b: ΔL, Δa, and Lb data generated from the L*a*b* data in Table 2a.**

| **Time (months)** | **ΔL** | **Δa** | **Δb** |
|---|---|---|---|
| **0** | 0.00 | 0.00 | 0.00 |
| **3** | 3.66 | 6.88 | 1.98 |
| **5** | -2.92 | 0.91 | -1.14 |
| **7** | 3.21 | 14.90 | 6.01 |
| **9** | 8.98 | 45.76 | 14.83 |

### Comparative Example 3

Below is an example of a generic formulation outside of the scope of the claims. The single content in the single compartment is formulated to be at a more basic pH (8.4-9.0) so to optimally deliver nicotine to a user. In the same content is a pH-sensitive component that is a flavour mix, that is incompatible with the pH of the single content.

### Single content in single compartment

| Material | Approx %w/w values | |
|---|---|---|
| | Lower range | Upper range |
| Nicotine | 0 | 1.6 |
| Solvent | 35 | 50 |
| Bulking Agent 1 | 15 | 32 |
| Bulking Agent 2 | 15 | 25 |
| Bulking Agent 3 | 0 | 15 |
| Humectant 1 | 4 | 10 |
| Humectant 2 | 0 | 3 |
| Buffering Agent | 0.1 | 0.6 |
| Flavour mix | 2 | 14 |

### Experimental Methods

L*a*b* measurements are taken using a PCE-XXM 20 colourimeter. The technical specifications of the instrument are as follows. Colour space: CIELAB CIEXYZ RGB, Pantone, CMYK. Colour difference formula: ΔE*Lab (CIE76/CIE94). Measuring aperture: diameter 8mm. Measuring geometry: 45°/diffused illumination. Type of light source: LED. Sensor: RGB silicon photoelectric diode. Viewing angle: CIE 10°Standard angle. Measurement interval: 1.5 s. Battery: Li-ion battery, chargeable via USB. Operating conditions: 0 ... 40 °C, < 80%. Storage conditions: 10 ... 30°C, < 70%. Measurements were taken of the whole smokeless article and at room temperature (≈20°C), including the water-permeable layer.

## Claims

1. A smokeless article for oral delivery of a nicotinic component, comprising: a water-permeable layer defining at least a first compartment and a second compartment; wherein the first compartment encloses a first content having a first pH, and the second compartment encloses a second content having a second pH; and wherein the first and second compartments are separated by a barrier which prevents passage of the first and second content between the first and second compartments; wherein the first pH is different to the second pH; and wherein at least one of the first and second content comprises a nicotinic component.

2. The smokeless article of claim 1, wherein the first content comprises the nicotinic component and the second content does not comprise the nicotinic component.

3. The smokeless article of claim 1 or claim 2, wherein the pH of the first content is in the range 7.5 to 10.

4. The smokeless article of claim 3, wherein the pH of the first content is in the range 8.4 to 9.

5. The smokeless article of any one of claims 1 to 4, wherein the pH of the second content is in the range 4 to 7.5.

6. The smokeless article of any one of claims 1 to 5, wherein the pH of the first content and the pH of the second content are different by at least 0.5 pH units.

7. The smokeless article of any one of claims 1 to 6, wherein the first and/or second content each independently contain a pH buffer.

8. The smokeless article of any one of claims 1 to 7, wherein the second content comprises a pH sensitive component that is incompatible with the pH of the first content, or the first content comprises a pH sensitive component that is incompatible with the pH of the second content.

9. The smokeless article of any one of claims 1 to 7, wherein the second content comprises a pH sensitive component that is compatible with the pH of the second content, or the first content comprises a pH sensitive component that is compatible with the pH of the first content.

10. The smokeless article of any one of claims 2 to 7, wherein the second content comprises a pH sensitive component that is incompatible with the pH of the first content.

11. The smokeless article of any one of claims 8 to 10, wherein the pH sensitive component comprises one or more of a flavouring, salt, a humectant, an active ingredient, a filler, a sweetener, a stabiliser and a binder.

12. The smokeless article of claim 11, wherein the pH sensitive component comprises a flavouring.

13. The smokeless article of any one of claims 1 to 12, wherein the barrier comprises a weld between two or more surfaces of the water-permeable layer.

14. A method of manufacturing the smokeless article of any one of claims 1 to 13, comprising: laser cutting the water-permeable layer to form shaped elements for forming the smokeless article; and thermoforming to seal the shaped elements to form the smokeless article.

15. A kit comprising a plurality of smokeless articles according to any one of claims 1 to 13.

16. Use of a smokeless article according to any one of claims 1 to 13, to formulate a pH sensitive component in a smokeless article.
